(19) Europäisches Patentamt
European Patent Office
Office européen des brevets

(11) Publication number: **0 129 863**
**B1**

(12) # EUROPEAN PATENT SPECIFICATION

(45) Date of publication of patent specification: **05.08.87**

(51) Int. Cl.⁴: **C 07 C 17/20,** C 07 C 19/08

(21) Application number: **84107156.6**

(22) Date of filing: **22.06.84**

(54) **Fluorination process.**

(30) Priority: **23.06.83 US 507084**

(43) Date of publication of application:
**02.01.85 Bulletin 85/01**

(45) Publication of the grant of the patent:
**05.08.87 Bulletin 87/32**

(84) Designated Contracting States:
**BE DE FR GB IT LU NL**

(56) References cited:
**DE-C- 573 534**
**US-A-2 005 705**
**US-A-2 005 708**
**US-A-2 005 711**
**US-A-2 510 872**

**Houben-Weyl, Bond V/3 (1962), 124-126**

(73) Proprietor: **E.I. DU PONT DE NEMOURS AND COMPANY**
**1007 Market Street**
**Wilmington Delaware 19898 (US)**

(72) Inventor: **Mader, Frederick William**
**12 Penn Oak Lane**
**Kennett Square Pennsylvania 19348 (US)**

(74) Representative: **Abitz, Walter, Dr.-Ing. et al**
**Abitz, Morf, Gritschneder, Freiherr von Wittgenstein Postfach 86 01 09**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 129 863 B1

Courier Press, Leamington Spa, England.

**Description**

Field of invention

This invention relates to a continuous process for manufacturing fluorinated derivatives of alkanes.

Background of the invention

Most commercial methods for producing fluorine-containing hydrocarbons are based on the reaction wherein hydrogen fluoride and haloalkanes containing halogen other than fluorine are reacted in the presence of antimony chloride catalysts (see, for example, U.S. 2,005,705 and U.S. 2,005,708). The reaction is that of replacing a nonfluorine halogen (Cl, Br or I) of the haloalkane with fluorine of the hydrogen fluoride. Most generally, the haloalkanes are chloroalkanes because of their availability and their tendency to undergo fewer side reactions during the exchange reaction than their corresponding bromo or iodo analogues.

The above-mentioned fluorination reaction may be represented by the following equation using chloroform as the illustrative haloalkane:

$$xHF + CHCl_3 \xrightarrow{\text{Antimony Chloride Catalyst}} CHCl_{3-x}F_x + xHCl \qquad (I)$$

where x is 1—3. Generally, a mixture of fluorinated products represented by $CHCl_{3-x}F_x$ is obtained which contains $CHCl_2F$, $CHClF_2$ and $CHF_3$, the particular proportion of these components depending upon the reactant ratios and the reaction conditions. Usually hydrogen fluoride is used in excess to insure maximum utilization of the haloalkane reactant. Therefore, the crude reaction product from the reaction will contain HF as well as a mixture of $CHCl_2F$, $CHClF_2$, $CHF_3$ and HCl. It will be noted from the above equation that for each mole of hydrogen fluoride undergoing the exchange reaction, one mole equivalent of hydrogen chloride is generated. The process is usually carried out continuously by cofeeding hydrogen fluoride and haloalkane into antimony chloride catalyst. Usually, a small amount of chlorine is also added to generate and to maintain the antimony in the pentavalent state.

The production of chlorofluoroalkanes by reaction of a haloalkane with hydrogen fluoride in the presence of antimony pentachloride catalyst has been used commercially for many years. However, as energy costs increase, the process becomes correspondingly more expensive and less attractive. For example, in the fluorination of chloroform the crude reaction products include $CHCl_2F$ (b.p. 8.9°C), $CHClF_2$ (b.p. −40.8°C), $CHF_3$ (b.p. −82°C), HCl (b.p. −85°C) and HF (b.p. 19.4°C). These reaction products must be separated from each other by cost-effective means. The conventional method of separation has been by a series of distillations which require a lot of energy for refrigeration and a high investment in pressurized equipment. Also, closeness in the boiling points of $CHF_3$ (−82°C) and HCl (−85°C) add to the difficulty and cost of the separation.

It is therefore an object of the present invention to provide an economical, energy efficient, continuous process for the production of fluorinated haloalkanes. It is a further object of the present invention to provide a continuous haloalkane fluorination process which will provide crude fluorinated products substantially free of hydrogen halides. It is a still further object of the present invention to provide a haloalkane fluorination process which minimizes costs associated with the separation of reaction products. These and other objects will be apparent from the description provided herein.

Definition of the invention

The present invention provides a continuous process for fluorinating haloalkanes containing at least one nonfluorine halogen atom comprising,

1) contacting antimony pentachloride with hydrogen fluoride in a first zone to replace at least a portion of chlorine in said antimony pentachloride with fluorine,

2) transferring the thus fluorinated antimony pentachloride to a second zone wherein the fluorination of antimony pentachloride to antimony chlorofluoride is essentially completed and hydrogen chloride generated in said fluorination of antimony pentachloride is separated from the antimony chlorofluoride,

3) transferring the antimony chlorofluoride to a third zone wherein said antimony chlorofluoride is contacted with said haloalkane to replace at least a portion of the nonfluorine halogen atoms of said haloalkane with fluorine,

4) removing spent antimony chlorofluoride from said third zone into a fourth zone wherein said spent antimony chlorofluoride is freed of volatile material and then recycled to the first zone for refluorination with hydrogen fluoride and

5) removing fluorinated haloalkanes from said third zone to a separation means to recover fluorinated haloalkanes essentially free of hydrogen halide.

Detailed description of the invention

Reference is made to the attached drawing for a more detailed explanation of the invention using

chloroform as the starting haloalkane reactant. Antimony pentachloride is introduced into first zone *A* via lines *1* and *3* while hydrogen fluoride is introduced into zone *A* via lines *2* and *3*. The hydrogen fluoride reacts with the antimony pentachloride in zone A replacing a portion of the chlorine atoms with fluorine. The reaction in this first zone can be carried out at temperatures ranging from about 60°C to about 140°C. A preferred temperature range is from about 70°C to about 120°C, while a temperature between about 90°C and 100°C is most preferred.

The chemical reaction taking place in zone *A* may be represented by the following equation:

$$SbCl_5+xHF \rightarrow SbCl_{5-x}F_x+xHCl \quad \text{(II)}$$

The reaction mixtures from zone *A* is transferred via line *4* to the second zone *B* which is a degassing vessel maintained at essentially the same temperature as the first zone. The reaction of hydrogen fluoride with antimony pentachloride proceeds to completion in zone *B* if it is not already completed in zone *A*. Hydrogen chloride formed in the fluorination of the antimony pentachloride is removed from zone *B* via scrubber *5* and exit line *6*. The introduction of antimony pentachloride via line *15* to scrubber *5* in a countercurrent fashion at juncture *16* assists in consuming any unreacted hydrogen fluoride that would otherwise be removed along with hydrogen chloride.

The antimony chlorofluoride in zone *B* is transferred via line *7* to a third zone *C*, the haloalkane reactor, wherein it is contacted with chloroform introduced into *C* via line *8*. The haloalkane reactor *C* is operated in the temperature range of from about 60°C to 150°C, preferably from about 75°C to about 125°C. Chlorine may also be introduced into *C* via line *9* to maintain the antimony in the pentavalent state. The chemical reaction proceeding in *C* may be represented by the following equation:

$$SbCl_{5-x}F_x+CHCl_3 \rightarrow CHCl_{3-x}F_x+SbCl_5 \quad \text{(III)}$$

wherein $CHCl_{3-x}F_x$ represents a mixture of mono-, di- and trifluorinated products. It will be noted from the above reaction that there are no hydrogen halides to separate from the fluorinated products.

The spent antimony chlorofluoride in zone *C* (i.e., regenerated antimony pentachloride) is passed via line *10* into a fourth zone *D* which is a degassing vessel wherein any unreacted haloalkane and/or fluorinated haloalkanes in the spent antimony chlorofluoride are allowed to separate from the spent antimony chlorofluoride and recycled to the haloalkane reactor *C* via line *11*. Such separation may be facilitated by methods well-known in the art such as by reduction in pressure or by heating.

The spent antimony chlorofluoride in zone *D* is then transferred via line *12* and pump *13* back to the first zone *A*. To alleviate contamination of the antimony pentachloride catalyst by arsenic compounds which are introduced into the system as impurities in the hydrogen fluoride, a small portion of the spent antimony chlorofluoride exiting from the pump *13* may be removed via line *14* and a corresponding amount of fresh antimony pentachloride may be introduced into the system via line *1*.

The fluorinated products formed in the haloalkane reactor *C* which include dichloromonofluoromethane, monochlorodifluoromethane and trifluoromethane are transferred via line *17* to a distillation column *E* wherein the desired fluorinated products are separated. In contrast to prior art processes, the fluorinated products are essentially free of hydrogen halides, and therefore, the separation of the fluorinated products by distillation is easier and much less costly. The manner in which the distillation column is operated depends upon the particular component of the product mixture desired. For example, if chloroform is being fluorinated with the objective of producing monochlorodifluoromethane as the chief end product, it would be recovered via line *20* while dichloromonofluoromethane and trifluoromethane would be recycled to the haloalkane reactor *C* via lines *18* and *19*, the former to be fluorinated further and the latter used to assist in stripping the fluorinated products from the antimony chlorofluoride. Higher boiling materials such as unreacted chloroform may also be recycled to *C* via line *19*. When excessive amounts of trifluoromethane build up in the system, they may be recovered via line *21*. The fluorinated haloalkanes produced by the process are useful as refrigerants, aerosol propellants, solvents and intermediates.

The hydrogen fluoride used to convert antimony pentachloride to antimony chlorofluoride may be a pure product or a commercial grade of hydrogen fluoride which may contain small amounts of impurities. The presence of water in the reactants is not desirable, and therefore it is preferable to use substantially anhydrous hydrogen fluoride. The hydrogen fluoride may be either gaseous or liquid hydrogen fluoride.

The antimony chloride used is generally in the pentavalent form, but it may contain up to about 10% of the antimony in the trivalent form. In order to maintain a desired high level of pentavalent antimony, chlorine gas may be introduced into the reactor to reoxidize any trivalent antimony formed by the action of reducing compounds present in the system.

The fluorination of antimony pentachloride with hydrogen fluoride may be carried out in the temperature range of from about 60°C to 140°C, preferably in the range of from about 70°C to 120°C, most preferably in the range of from about 90°C to about 120°C. The pressure may be atmospheric, subatmospheric or superatmospheric. It is preferred to use superatmospheric pressures, particularly when the fluorination of antimony pentachloride is carried out in the higher temperature ranges indicated. The degree of fluorination of antimony pentachloride may be varied over a wide range such that fluorinated

antimony chlorides contain from about 2% to about 40% by weight of fluorine. It is preferred that the fluorine content be in the range from about 3% to about 10% for ease of handling and to minimize the corrosivity of the fluorinated antimony pentachloride towards certain metals. Since the fluorinated antimony chlorides are liquids at ordinary temperatures, particularly when the trivalent antimony does not exceed about 10%, they may be transferred to the degassing vessel *B* and subsequently to haloalkane reactor *C* using the pressure generated by the hydrogen chloride formed in the fluorination of the pentavalent antimony chloride in vessel *A*, or by introduction of a pressurized inert gas such as nitrogen. It is preferred to use hydrogen chloride since the use of other gases, such as nitrogen, will necessitate their separation at a later stage of the operation. The use of a mechanical pump to move fluorinated antimony chloride is to be avoided because of the corrosive nature of the fluorinated antimony chloride towards materials of construction.

The degasser *B* is kept at a temperature approximating that of the antimony pentachloride fluorinator there being no requirement to cool or heat the antimony chlorofluoride in the degasser although, if desired, heating may be used. The pressure in the degasser *B* should be less than the pressure in the antimony pentachloride fluorinator *A* in order to transfer the fluorinated antimony chloride from the fluorinator to the degasser using pressure differentials and to facilitate the separation of hydrogen halide from the fluorinated antimony chloride. The fluorinated antimony chloride is kept in the degasser for such time as to complete the reaction of hydrogen fluoride with antimony pentachloride and to essentially separate the by-product hydrogen chloride from the reaction mass. Usually the reaction of hydrogen fluoride and the desorption of by-product hydrogen chloride is rapid enough that a few minutes residence in the degasser is sufficient to accomplish these two objectives.

The hydrogen chloride separated at the degasser is ordinarily free of hydrogen fluoride but as an extra precaution against hydrogen fluoride contamination, the exiting hydrogen chloride stream can be contacted with fresh antimony pentachloride or with spent antimony chlorofluoride which will react with hydrogen fluoride present. The hydrogen chloride can be collected as anhydrous hydrogen chloride or dissolved in water to form hydrochloric acid.

Contact between reactants in the haloalkane fluorination reactor *C* can be accomplished by conventional mixing methods such as by countercurrent flow, back mixing, etc. The temperature in this fluorinator is usually maintained in the range of from about 60°C to about 150°C, preferably from about 70°C to about 120°C. As previously mentioned, the pressure may be atmospheric, subatmospheric or superatmospheric, but generally it is preferred to use superatmospheric pressures of up to about 20 atmospheres, particularly when the haloalkane to be fluorinated is a one carbon atom haloalkane. An important consideration in the choice of pressure is that it is preferable to use a pressure that will maintain the antimony halide in the liquid phase while allowing vaporization of fluorinated haloalkanes.

The temperature employed, the mol ratio of haloalkane to antimony chlorofluoride, and the contact time between the antimony chlorofluoride and the haloalkane used will depend upon the degree of fluorination of the antimony pentachloride and the end product that is desired. Generally, a combination of a high level of haloalkane with a low degree of fluorination of antimony pentachloride will favor monofluorination whereas a combination of a low level of haloalkane with a high degree of fluorination in the antimony pentachloride will favor multiple fluorination. In either case, the fluorinated haloalkanes obtained will be a mixture of fluorinated compounds. The notable feature of the present invention is that in contrast to the prior art processes, these fluorinated haloalkanes are produced without the coproduction of equivalent amounts of hydrogen halide. If the antimony chlorofluoride is thoroughly degassed of hydrogen halide before introduction into the haloalkane fluorination reactor, the fluorinated haloalkane produced will be completely free of hydrogen halide. Generally, the amount of hydrogen halide present in the crude fluorinated haloalkane will be considerably less than 5% of that obtained in prior art processes where haloalkane is reacted with hydrogen fluoride in the presence of antimony halide catalysts. The present invention thus provides the advantages of producing crude fluorinated haloalkane substantially free of hydrogen fluoride which makes the recovery of fluorinated haloalkanes considerably easier and more economical by eliminating a number of distillation systems which would have required high pressures and costly refrigeration and at the same time provides commercially valuable hydrogen chloride as a by-product.

The term "haloalkane" is meant to include those alkanes substituted with at least one halogen atom other than a fluorine atom, the halogen atoms being those of chlorine, bromine and iodine. The haloalkane may also contain fluorine atoms provided there is present at least one nonfluorine halogen atom. The term "haloalkane" is also intended to include those combinations of olefins and halogens which theoretically form haloalkanes during reaction; for example, a combination of trichloroethylene and chlorine or a combination of tetrachloroethylene and chlorine. The preferred haloalkanes are those wherein the halogen substituent is chlorine since they are lower in cost and readily available and have less tendency to undergo side reactions in the fluorinator such as rearrangement reactions. Theoretically, any haloalkane which can be readily introduced into the haloalkane fluorinator can be used in the present process, but haloalkanes containing from about 1 to 3 carbon atoms are usually employed. Preferred haloalkanes are those containing 1 to 2 carbon atoms while those containing one carbon atom are most preferred. The preferred haloalkanes of two carbon atoms are pentachloroethane and hexachloroethane. These haloalkanes may be directly supplied to the haloalkane fluorination reactor, or they may be supplied by the introduction of a

combination of trichloroethylene and chlorine or a combination of tetrachloroethylene and chlorine to the haloalkane fluorinator. These combinations respectively form pentachloroethane and hexachloroethane in situ. The preferred one carbon atom haloalkanes are chloroform and carbon tetrachloride.

The following examples further illustrate the invention. Unless otherwise specified, all percentages are by weight.

Example 1

Fluorination of antimony pentachloride with liquid hydrogen fluoride was carried out in a "Teflon" polytetrafluoroethylene cylinder of 115 ml capacity. Antimony pentachloride (70.6 g) was placed in the cylinder and the cylinder was connected to a hydrogen fluoride cylinder. The antimony pentachloride in the "Teflon" cylinder was frozen by packing the lower end of the cylinder in solid carbon dioxide. After the antimony pentachloride was frozen, the cylinder was evacuated and hydrogen fluoride (49.0 g) was transferred to the "Teflon" cylinder by distillation from the hydrogen fluoride cylinder. After disconnection from the hydrogen fluoride cylinder, the "Teflon" cylinder was connected to a drying tube which, in turn, was connected to a gas bubbler containing liquid trichlorotrifluoroethane to observe the evolution of hydrogen chloride. The "Teflon" cylinder was maintained at 8—12°C overnight after which time the evolution of hydrogen chloride ceased. The contents of the cylinder was purged with dry nitrogen and then placed under vacuum to remove any volatile material. The residue in the cylinder weighed 66.6 g which compares with the theoretical weight of 66.7 g wherein one of the five chlorides of the antimony pentachloride is replaced by fluorine. The lower outside portion of the "Teflon" cylinder was again packed in solid carbon dioxide and the cylinder again evacuated. Carbon tetrachloride (29.6 g) was distilled into the "Teflon" cylinder and the contents of the cylinder was allowed to warm to room temperature. The mol ratio of the antimony chlorofluoride to carbon tetrachloride was 1.22. The outlet of the cylinder was connected to a water scrubber which, in turn, was connected to a receiver placed in solid carbon dioxide-methanol bath. The cylinder was heated in an oil bath starting at 70°C and up to 80°C over a two-hour period. The material collected in the cold receiver weighed 28.5 g. Analysis of the organic material in the receiver based upon area percent from vapor phase chromatography indicated 0.3% monochlorotrifluoromethane, 16.74% dichlorodifluoromethane, 81.3% trichloromonofluoromethane and 1.42% carbon tetrachloride. Analysis of the spent antimony chlorofluoride fluorinating agent indicated that 71.5% of the fluorine in the fluorinating agent was utilized (from 7.0% fluorine down to 1.95% fluorine).

Example 2

Antimony chlorofluoride was prepared as described in Example 1 except that a stainless steel cylinder was employed in place of the "Teflon" cylinder, and 82.6 g of antimony pentachloride and 62 g of liquid hydrogen fluoride were used.

Antimony chlorofluoride (76.5 g) was reacted with 40 g of carbon tetrachloride (mol ratio of fluorinating agent to carbon tetrachloride of 1.06) at 85°C—90°C for twenty hours. Pressure of 118 psig at 88°C was developed. After cooling, the cylinder was connected to a water scrubber and thence to a receiver placed in a solid carbon dioxide-methanol bath. The cylinder was heated to 42°C and 15.37 g of product was collected in the receiver. Analysis of the organic material in the receiver based on area percent from vapor phase chromatography indicated the products to be 0.36% monochlorotrifluoromethane, 43.94% dichlorodifluoromethane, 49.77% trichloromonofluoromethane and 4.76% carbon tetrachloride. Analysis of the spent antimony chlorofluoride fluorinating agent indicated that 94% of the fluorine in the fluorinating agent was utilized (Initial fluorine content: 9.34%. Final fluorine content: 0.56%).

Example 3

Antimony chlorofluoride was prepared as described above using 82.8 g antimony pentachloride and 60.8 g hydrogen fluoride. The halocarbon reacted was hexachloroethane (17 g) which was added as a solid to the antimony chlorofluoride. The mol ratio of antimony chlorofluoride to hexachloroethane was 3.86. The reaction was carried out at 145°C—160°C for 20 hours during which time pressure of 65 psig at 146°C developed (after 14.5 hours). On cooling the cylinder to 23°C, the pressure dropped to 20 psig. Using the above-described collection procedure, 6.27 g of material was collected in the receiver which material based on area percent from vapor phase chromatography was indicated to be 0.18% dichlorotetrafluoroethane, 71.77% trichlorotrifluoroethane, 16.05% tetrachlorodifluoroethane, 0.48% penta-chloromonofluoroethane, 9.57% trichloromonofluoromethane and 1.72% carbon tetrachloride. Analysis of the spent antimony chlorofluoride fluorination reagent indicated that 46.4% of the fluorine in the antimony chlorofluoride was utilized (Initial fluorine content: 10.45%. Final fluorine content: 5.6%).

Example 4

Antimony chlorofluoride was prepared as previously described from 81.6 g antimony pentachloride and 53.8 g hydrogen fluoride. Chloroform, 24.6 g, was added to the antimony chlorofluoride and the mixture heated to 75°C—80°C for 17 hours. The mol ratio of antimony chlorofluoride to chloroform was 1.32. The pressure in the cylinder reached 186 psig at 78°C. On cooling to 35—40°C, the pressure dropped to around 122 psig. On venting through a water scrubber and a receiver kept in solid carbon dioxide-methanol bath, there was obtained 4.88 g of products in the cooled receiver. Analysis of the product based on area

percent obtained in vapor phase chromatography indicated 10.16% trifluoromethane, 85.46% monochlorodifluoromethane, 2.24% dichloromonofluoromethane and 2.01% chloroform. Analysis of the spent antimony chlorofluoride fluorinating agent indicated that 80.2% of the fluoride in the antimony chlorofluoride was utilized (Initial fluorine content: 7.44%. Final fluorine content: 1.47%.)

Example 5

790 g (2.64 moles) of antimony pentachloride was placed in a 1-liter stainless steel reaction vessel equipped with a condenser with ice water coolant. The vessel was heated to 90°C and 52 g (2.6 moles) of gaseous hydrogen fluoride was bubbled through the antimony pentachloride over a 2-hour period. After the addition of hydrogen fluoride was stopped, the content of the vessel was purged with nitrogen four times, allowing the content to come to an equilibrium for about an hour between purges. Fluorine analysis indicated that the antimony chlorofluoride contained 6.35% by weight of fluorine.

Example 6

227 g (0.8 mole) of antimony chlorofluoride prepared in Example 5 was placed in a 1-liter stainless steel vessel equipped with a stirrer, and 93 g (0.6 mole) of carbon tetrachloride was added over a period of 40 minutes with the stirrer in operation. The reaction vessel temperature ranged from 80°C to 132°C. The volatile material evolved during the carbon tetrachloride addition was sampled at various times during the 40 minutes reaction time. Total volume of gas evolved was 9 liters. Gas chromatographic analyses of the samples, the time of reaction and the temperatures are summarized in the following table:

| Time (minutes) | Temperature (°C) | Area* percent | | | |
|---|---|---|---|---|---|
| | | $CCl_3F$ | $CCl_2F_2$ | $CClF_3$ | $CCl_4$ |
| 8 | 120 | 77.88 | 12.57 | 2.83 | 6.71 |
| 12 | 132 | 50.77 | 39.33 | 5.46 | 4.43 |
| 16 | 120 | 46.91 | 44.16 | 4.55 | 4.38 |
| 24 | 86 | 35.81 | 58.79 | 2.48 | 2.92 |
| 34 | 80 | 49.31 | 46.79 | 1.25 | 2.66 |

* Area percent is determined by dividing the area of the chromatographic peak for a particular component by the total area of all peaks.

The fluorine content of the antimony chlorofluoride dropped from 6.35% to 0.11%. The hydrogen chloride content in the fluorinated product was 7.03% whereas the theoretical amount of hydrogen chloride evolved in the prior art process of cofeeding hydrogen fluoride and carbon tetrachloride to the antimony pentachloride catalyst is 38%. The presence of 7.03% hydrogen chloride in the product is probably due to incomplete removal of hydrogen chloride from antimony chlorofluoride or the presence of hydrogen fluoride in the antimony chlorofluoride.

Example 7

229 g (0.8 mole) of antimony chlorofluoride prepared in Example 5 was placed in a 1-liter stainless steel vessel equipped with a stirrer, and 45 g (0.38 mole) of chloroform was added over a 40-minute period with the stirrer in operation. The reaction vessel temperature ranged from 100°C to 108°C. Volatile material evolved during the reaction was sampled at various times and analyzed. Total volume of gas evolved was 5.4 liters. Gas chromatographic analyses of the fluorinated products, the time of reaction and the temperatures are summarized below:

| Time (minutes) | Temperature (°C) | Area percent | | |
|---|---|---|---|---|
| | | $CHCl_2F$ | $CHClF_2$ | $CHF_3$ |
| 12 | 104 | 4.67 | 64.13 | 31.20 |
| 18 | 100 | 5.09 | 43.4 | 51.71 |
| 27 | 105 | 27.23 | 31.61 | 41.15 |
| 36 | 108 | 35.44 | 57.40 | 6.80 |

The spent antimony chlorofluoride contained 2.09% fluorine (Initial fluorine content: 6.35%.) The

hydrogen chloride content in the fluorinated product was 3.8% whereas the theoretical amount of hydrogen chloride evolved in the prior art process of cofeeding hydrogen fluoride and chloroform to the antimony pentachloride catalyst is 46%.

Example 8

This example illustrates a continuous reaction of chloroform with antimony chlorofluoride in a packed tower wherein chloroform gas is contacted countercurrently with antimony chlorofluoride. 1742 g antimony chlorofluoride (5.05% fluorine) at 85°C to 90°C prepared as described in Example 5 was added continuously through a 1/4 inch stainless steel line to the top of a 2.5 cm×56 cm stainless steel tube packed with 6.35 mm extended stainless steel packing ("Pro-Pak"). 1130 g of chloroform gas, at 80°C was fed upward from the bottom end of the stainless steel tube and contacted with the downward flowing antimony chlorofluoride. The contact time was from 2 to 40 seconds. Spent antimony chlorofluoride was collected in a vessel connected to the lower end of the reaction tube. The volatile reaction product passed from the reaction tube through a cooled condenser, a trap, two water scrubbers, gas sampling bulbs, and finally through a wet test meter. The chloroform was introduced over a 250-minute period. The temperature in the reactor tube ranged from 50°C to 90°C. Gas samples at 35, 60, 100, 120, 180, 210, 240 and 250 minutes were collected and analyzed by gas chromatography. The average values of these 8 analyses reported as area percentages indicated that the volatile products consisted of trifluoromethane 0.6% (range of 0.24 to 0.86%), monochlorodifluoromethane 10.5% (range of 1.02 to 21.83), dichloromonofluoromethane 83.47% (range of 73.21 to 93.27%), chloroform 3.1% (range of 0.96 to 4.48%), trichloromonofluoromethane 1.54% (range of 0.02 to 5.98%), dichlorodifluoromethane 0.25% (range of 0.09 to 0.62%). The conversion of chloroform was 32%. About 93% of the available fluorine in the antimony chlorofluoride was utilized (Initial fluorine content: 5.05%. Final fluorine content: 0.33%.)

## Claims

1. A continuous process for fluorinating haloalkanes comprising

continuously fluorinating antimony pentachloride to antimony chlorofluoride by supplying continuous streams of said antimony pentachloride and hydrogen fluoride to a reaction zone while continuously removing by-product hydrogen chloride gas from the antimony chlorofluoride thus produced,

continuously transferring the separated antimony chlorofluoride to a separate reaction zone while supplying a haloalkane containing at least one nonfluorine halogen atom to said separate reaction zone and reacting the antimony chlorofluoride with said haloalkane thereby replacing a portion of the nonfluorine halogen in said haloalkane with fluorine of the antimony chlorofluoride and recovering the chlorofluoroalkane reaction products produced.

2. The process of Claim 1 in which the haloalkane is chloroform.

3. The process of Claim 1 in which spent antimony chlorofluoride from the reaction with the haloalkane is recycled to the reaction zone where hydrogen fluoride is supplied.

4. The process of Claim 3 in which the haloalkane is chloroform.

5. The process of Claim 3 in which the chlorofluoroalkane reaction products are recovered by fractional distillation.

6. The process of Claim 5 in which the haloalkane is chloroform.

## Patentansprüche

1. Kontinuierliches Verfahren zur Fluorierung von Halogenalkanen, bei dem

Antimonpentachlorid kontinuierlich zu Antimonchlorfluorid fluoriert wird, indem man kontinuierliche Ströme von Antimonpentachlorid und Fluorwasserstoff einer Reaktionszone zuführt, während man das als Nebenprodukt gebildete Chlorwasserstoffgas kontinuierlich aus dem so hergestellten Antimonchlorfluorid entfernt,

kontinuierlich das abgetrennte Antimonchlorfluorid in eine getrennte Reaktionszone überführt wird, während man ein Halogenalkan, das mindestens ein Nichtfluor-Halogenatom enthält, der getrennten Reaktionszone zuführt und das Antimonchlorfluorid mit dem genannten Halogenalkan umsetzt, wodurch ein Teil des Nichtfluor-Halogens in dem Halogenalkan mit Fluor aus dem Antimonchlorfluorid werdrängt wird und die gebildeten Chlorfluoralkan-Reaktionsprodukte gewonnen werden.

2. Verfahren nach Anspruch 1, bei dem das Halogenalkan Chloroform ist.

3. Verfahren nach Anspruch 1, bei dem bei der Reaktion mit dem Halogenalkan verbrauchtes Antimonchlorfluorid im Kreislauf der Reaktionszone wieder zugeführt wird, in die Fluorwasserstoff eingespeist wird.

4. Verfahren nach Anspruch 3, bei dem das Halogenalkan Chloroform ist.

5. Verfahren nach Anspruch 3, bei dem die Reaktionsprodukte der Chlorfluoralkanreaktion durch fraktionierte Destillation gewonnen werden.

6. Verfahren nach Anspruch 5, bei dem das Halogenalkan Chloroform ist.

**Revendications**

1. Un procédé continu pour fluorer des halogénoalcanes, qui comprend

la fluoration en continu de pentachlorure d'antimoine pour le transformer en chlorofluorure d'antimoine en envoyant des courants continus dudit pentachlorure d'antimoine et de fluorure d'hydrogène dans une zone de réaction tout en éliminant en continu du chlorofluorure d'antimoine ainsi produit le chlorure d'hydrogène gazeus obtenu comme sous-produit,

Le transfert en continu du chlorofluorure d'antimoine séparé dans une zone de réaction séparée tout en envoyant un halogénoalcane contenant au moins un atome d'halogène autre que le fluor dans ladite zone de réaction séparée, la réaction du chlorofluorure d'antimoine avec ledit halogènoalcane pour remplacer ainsi une partie de l'halogène autre que le fluor contenu dans ledit halogénoalcane par le fluor du chlorofluorure d'antimoine et le recueil des chlorofluoroalcanes obtenus comme produits réactionnels.

2. Le procédé de le Revendication 1, dans lequel l'halogénoalcane est le chloroforme.

3. Le procédé de la Revendication 1, dans lequel le chlorofluorure d'antimoine usé issu de la réaction avec l'halogénoalcane est recyclé dans la zone de réaction où est envoyé le fluorure d'hydrogène.

4. Le procédé de la Revendication 3, dans lequel l'halogénoalcane est le chloroforme.

5. Le procédé de la Revendication 3, dans lequel les chlorofluoroalcanes obtenus comme produits reactionnels sont recueillis par distillation fractionnée.

6. Le procédé de la Revendication 5, dans lequel l'halogénoalcane est le chloroforme.

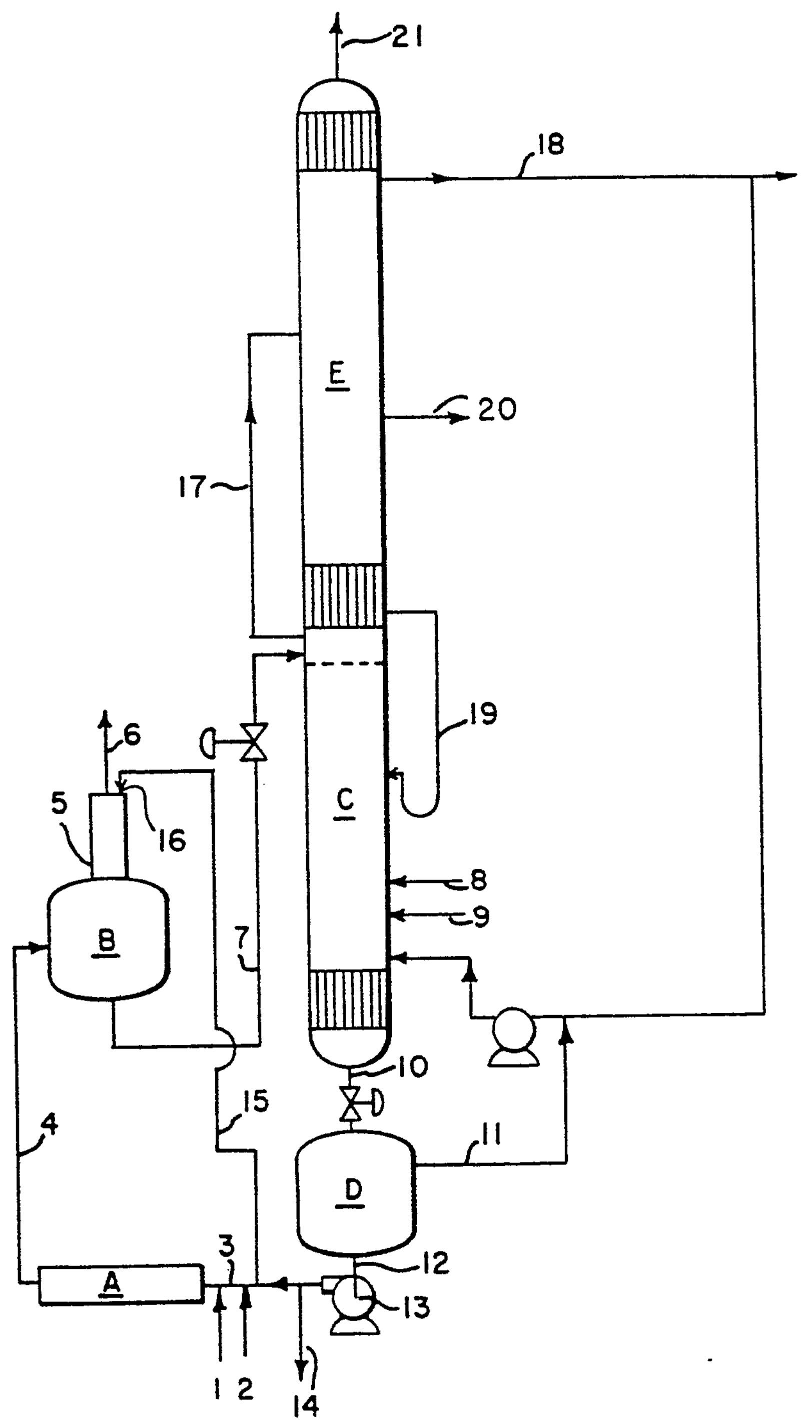
21
18
E
20
17
19
6
C
5
16
8
9
B
7
10
15
4
11
D
3
12
A
13
1 2
14